# EUROPEAN PATENT SPECIFICATION

(11) **EP 2 782 588 B1**
(45) Date of publication and mention of the grant of the patent: **15.04.2020**
(21) Application number: 12850971.8
(22) Date of filing: 17.11.2012
(51) Int. Cl.: A61K 38/00, A61K 31/198, A61P 3/10, A61P 3/06

(54) **METHODS AND COMPOSITIONS FOR THE TREATMENT OF DIABETES AND RELATED SYMPTOMS**
VERFAHREN UND ZUSAMMENSETZUNGEN ZUR BEHANDLUNG VON DIABETES UND ZUGEHÖRIGER SYMPTOME
PROCÉDÉS ET COMPOSITIONS POUR LE TRAITEMENT DU DIABÈTE ET DE SYMPTÔMES ASSOCIÉS

(30) Priority: 21.11.2011 US 201161629633 P
(43) Date of publication of application: 01.10.2014
(60) Divisional application: 18196395.0
(73) Proprietor: Emmaus Medical, Inc., Torrance, CA 90501 (US)
(72) Inventor: NIIHARA, Yutaka, Rolling Hills Estates, CA 90274 (US)
(74) Representative: Boult Wade Tennant LLP
(86) International application number: PCT/US2012/000557
(87) International publication number: WO 2013/077893

(56) References cited:
- WO-A1-03/072556
- WO-A1-2010/107307
- WO-A2-2011/038014
- US-A1- 2008 221 074
- Emmanuel C Opara ET AL: "L-Glutamine Supplementation of a High Fat Diet Reduces Body Weight and Attenuates Hyperglycemia and Hyperinsulinemia in C57BL/6J Mice1'2'3", , 1 January 1996 (1996-01-01), XP055182402, Retrieved from the Internet: URL:http://jn.nutrition.org/content/126/1/ 273.full.pdf#page=1&view=FitH [retrieved on 2015-04-13]
- CRISTIANO MASSAO TASHIMA ET AL: "Diabetic Rats Supplemented with L-Glutamine: A Study of Immunoreactive Myosin-V Myenteric Neurons and the Proximal Colonic Mucosa", DIGESTIVE DISEASES AND SCIENCES, KLUWER ACADEMIC PUBLISHERS-PLENUM PUBLISHERS, NE, vol. 52, no. 5, 28 March 2007 (2007-03-28) , pages 1233-1241, XP019499938, ISSN: 1573-2568, DOI: 10.1007/S10620-006-9564-8

## Description

### Field of the Invention

The present invention is directed to methods and compositions for the treatment of diabetes and related symptoms. It is more specifically directed to compositions including L-glutamine, its salts, or its derivatives, and uses of such compositions in the treatment of diabetes and related symptoms.

### Background of the Invention

Diabetes mellitus, also referred to as diabetes, is a group of metabolic diseases in which a person has high blood sugar levels. A person with diabetes either does not produce enough insulin to regulate blood sugar levels, or the person's cells do not respond properly to the insulin that is produced.

There are three primary types of diabetes: Type 1; Type 2; and, gestational diabetes. Type 1 diabetes results from the body's inability to produce insulin. Type 2 diabetes is the result of insulin resistance; a person's cells fail to use insulin properly. Gestational diabetes is experienced by certain women during pregnancy, where there was no prior history of Type 2 diabetes.

Diabetes is diagnosed through measurements obtained through one or more blood tests. For instance, the following tests and related values are typically indicative of diabetes: a fasting plasma glucose level ≥ 7.0 mmol/L (126 mg/dL); a plasma glucose level ≥ 11.1 mmol/L (200 mg/dL) two hours after a 75 g oral glucose load (*e*.*g*., glucose tolerance test); a casual plasma glucose level ≥ 11.1 mmol/L (200 mg/dL) in conjunction with symptoms of hyperglycemia; a glycolated hemoglobin (*i.e.,* Hb A1C) level ≥ 6.5%.

Symptoms of hyperglycemia can include, without limitation, excess thirst, fatigue, frequent urination, hunger, weight loss, and hypertriglyceridemia. A person has hypertriglyceridemia if his triglyceride blood levels are over 200 mg/dL; any value over 500 mg/dL is considered to be extremely high. Normal triglyceride levels are typically less than 150 mg/dL.

There is a need in the art for additional compositions and methods that can be used to treat diabetes and its related symptoms. That is an object of the present invention.

WO2011/038014 describes a method for treating incretin related diseases such as diabetes, obesity and the like by delivery by butyric acid, bile acid, long chain fatty acid or glutamine to the colon bypassing the upper digestive tract.

### Brief Description of the Figures

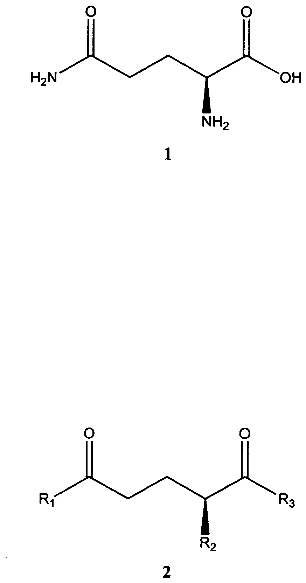

**FIG. 1** shows L-glutamine **(1)** and L-glutamine salts and derivative **(2).**

### Summary of the Invention

The invention is as defined in the appended claims. The present invention provides L-glutamine or an L-glutamine salt for use in a method of treating hypertriglyceridemia. The method involves ingesting 0.05 g/kg body weight to 10.0 g/kg body weight of L-glutamine, an L-glutamine salt of an L-gluatmine derivative per day by a person who has hypertriglyceridemia.

### Detailed Description of the Invention

The structure of L-glutamine is shown in **FIG. 1**, compound **1.** L-glutamine salts and derivatives are shown as compound **2** in **FIG. 1.** Nonlimiting L-glutamine salts and derivatives have the following substituents in reference to compound **2**:
R₁ is NH₂;
R₂ is NH₂, NH₃⁺, NH₃Br, NH₃OPO₃H, NH₃OC(O)CH₃ (*i.e*., acetate salt), NH₃OC(O)CHCHCO₂H (*i.e*., fumarate salt - *trans* olefin), NH₃OC(O)CH(OH)CH(OH)CO₂H (*i.e*., tartrate salt), NH₃OC(O)CHCHCO₂H (*i.e*., maleate salt - *cis* olefin), NH₃OC(O)CH₂-C(OH)(CO₂H)CH₂CO₂H (*i.e*., citrate salt), NH₃OC(O)CO₂H (*i.e*., oxalate salt), NH₃OS(O)₂OH (*i.e*., methanesulfonate salt), NH₃OS(O)₂C₆H₄CH₃ (*i.e*., p-toluenesulfonate salt), and, NH₃OC(O)C(O)CH₂CH₂CO₂H (*i.e*., alpha-ketoglutarate salt).
R₃ is OH, O⁻, ONa, OK, OCa, OLi, ONH₂(CH₂C₆H₅)CH₂CH₂NHCH₂C₆H₆ (*i.e*., benzathine salt), ON(CH₂CH₃)₂CH₂CH₂OC(O)C₆H₃ClNH₂ (*i.e*., chloroprocaine salt), ON(CH₃)₃CH₂CH₂OH (*i.e*., choline salt), ONH₂(CH₂CH₂OH)₂ (*i.e*.diethanolamine salt), ONH₃CH₂CH₂OH (*i.e*., ethanolamine salt), ONH₃CH₂CH₂NH₂ (*i.e*., ethyldiamine salt) ONH₂(CH₃)CH₂CH(OH)CH(OH)CH(OH)CH(OH)CH₂OH (*i.e*., meglumine salt), ONH₃C(CH₂OH)₃ (*i.e*., tromethamine salt), ONH₃C(CH₃)₃ (*i.e*., tertiary-butylamine salt) ON(CH₂CH₃)₂CH₂CH₂OC(O)C₆H₄NH₂ (*i.e*., procaine salt), NHCH(CH₃)CO₂H, NHCH(CH(CH₃)₂)CO₂H, NHCH(CH(CH₃)(CH₂CH₃))CO₂H, NHCH(CH₂CH(CH₃)₂)CO₂H, NHCH(CH₂CH₂SCH₃)CO₂H, NHCH(CH₂C₆H₅)CO₂H, NHCH(CH₂C₆H₅OH)CO₂H, NHCH(CH₂C₈H₆N)CO₂H, NHCH₂CO₂H, NHCH(CH₂CH₂C(O)NH₂)CO₂H, NHCH(CH₂C(O)NH₂)CO₂H, NHCH(CH(OH)CH₃)CO₂H, NHCH(CH₂OH)CO₂H, NHCH(CH₂CH₂CO₂H)CO₂H, NHCH(CH₂CO₂H)CO₂H.

Typically, a single compound (*e*.*g*., L-glutamine) is administered to a person seeking treatment for diabetes or its symptoms. In certain cases, however, a mixture of two or more compounds may be administered. For instance, L-glutamine may be administered with one or more L-glutamine salts or derivatives.

L-glutamine, its salts, derivatives or mixtures may be administered in any suitable way. For example, in certain cases it is administered as an aqueous solution. The solution may only have L-glutamine, salts or derivatives as dissolved ingredients, or it may include other pharmaceutically acceptable compounds. Nonlimiting examples of such compounds include buffers and flavorants.

Other, non-limiting examples of ways in which L-glutamine, its salts, derivatives or mixtures may be administered include: as a suspension in a liquid (*e*.*g*., water or juice); as a solid, either as a powder or in another form (*e.g*., pill or capsule); as a mixture with food (*e.g*., yogurt). As with the aqueous solution, the preceding administration forms may include other pharmaceutically acceptable ingredients.

The amount of L-glutamine, salts or derivatives administered according to the present invention typically range from 0.05 g/kg body weight to 10.0 g/kg body weight. Oftentimes, the amount ranges from 0.10 g/kg body weight to 8.0 g/kg body weight. In certain cases, the amount ranges from 0.15 g/kg body weight to 7.0 g/kg body weight.

L-glutamine, its salts or derivatives are typically administered to a person once per day. The compounds may, however, be administered more than once per day where indicated.

Compositions described herein may be used to treat diabetes as determined by a number of different tests that measure blood sugar levels. After administration of L-glutamine, its salts or derivatives for at least one month, two months, or three months, the fasting plasma glucose level of a person who previously tested at a level ≥ 7.0 mmol/L is typically reduced to ≤ 6.9 mmol/L. Oftentimes, it is reduced to ≤ 6.8 mmol/L or ≤ 6.7 mmol/L.

The plasma glucose level two hours after a 75 g oral glucose load of a person who previously tested at a level ≥ 11.1 mmol/L is typically reduced to ≤ 11.0 mmol/L. Oftentimes the level is reduced to ≤ 10.9 mmol/L or ≤ 10.8 mmol/L. In certain cases, it is reduced to ≤ 10.7 mmol/L.

The glycolated hemoglobin level of a person who previously tested at a level ≥ 6.5% is typically reduced to ≤ 6.4%. Oftentimes the level is reduced to ≤ 6.3 % or ≤ 6.2%. In certain cases, it is reduced to ≤ 6.1%.

Compositions for use according to the present invention may also be used to treat certain symptoms of diabetes, including hypertriglyceridemia. After administration of L-glutamine, its salts or derivatives for at least one month, two months or three months, the triglyceride level of a person who previously tested at a blood level over 500 mg/dL is typically reduced to less than 200 mg/dL. In certain cases, the triglyceride blood level is reduced to less than 180 mg/dL or 160 mg/dL. The triglyceride level of a person who previously tested at a blood level over 200 mg/dL is typically reduced to less than 200 mg/dL or less than 190 mg/dL. Oftentimes, the triglyceride level is reduced to less than 180 mg/dL, 170 mg/dL, 160 mg/dL or 150 mg/dL.

Where compositions of the present invention are used to treat diabetes, they may be combined with other methods used to treat diabetes. For instance, the compositions may be administered in conjunction with insulin sensitizers, secretagogues and/or alpha-glucosidase inhibitors. Nonlimiting examples of insulin sensitizers include: biguanides (*e*.*g*., metformin (Glucophage)); thiazolidinediones (e.g., rosiglitazone (Avandia), pioglitazone (Actos)). Nonlimiting examples of secretagogues include: sulfonylureas (e.g., tolbutamide (Orinase), acetohexamide (Dymelor), tolazamide (Tolinase), chlorpropamide (Diabinese), glipizide (Glucotrol), glyburide (Diabeta, Micronase, Glynase), glimepiride (Amaryl), and gliclazide (Diamicron)); and nonsulfonylureas (*e*.*g*., repaglinide (Prandin) and nateglinide (Starlix)). Nonlimiting examples of alpha-glucosidase inhibitors include: miglitol (Glyset); and, acarbose (Precose/Glucobay).

Where compositions of the present invention are used to treat hypertriglyceridemia, they may be combined with other methods used to treat hypertriglyceridemia. For instance, the compositions may be administered in conjunction with statins, fibrates, niacin, fish oil, and prescription omega-3-acid ethyl esters. Nonlimiting examples of statins include: atorvastatin (Lipitor); fluvastatin (Lescol); lovastatin (Mevacor); pravastatin (Pravachol); rosuvastatin (Crestor); and, simvastatin (Zocor). Nonlimiting examples of fibrates include: fenofibrate (Tricor); and, gemfibrozil (Lopid). A nonlimiting example of prescription omega-3-acid ethyl esters include Omacor.

### Experimental Results

### HA1C Lowering

A person had a measured glycolated hemoglobin level (*i.e.,* Hb A1C level) of 6.9%. The person ingested 30 g of L-glutamine per day as an aqueous solution. After three months of L-glutamine administration, the person's Hb A1C level was measured at 6.1%.

### Tryglyceride Lowering

A person had a measured triglyceride blood level of 500 mg/dL. The person ingested 30 g of L-glutamine per day as an aqueous solution. After three months of L-glutamine administration, the person's triglyceride blood level was measured at 150 mg/dL.

## Claims

1. L-glutamine or an L-glutamine salt for use in treating hypertriglyceridemia, wherein 0.05 g/kg body weight to 10.0 g/kg body weight of the L-glutamine or the L-glutamine salt is ingested per day by a person having diabetes.

2. L-glutamine for use according to claim 1.

3. L-glutamine for use according to claim 2, wherein the L-glutamine is ingested as part of an aqueous solution.

4. L-glutamine for use according to claim 3, wherein the L-glutamine is ingested for a period of at least one month.

5. L-glutamine for use according to claim 4, wherein the person having hypertriglyceridemia, had a measured blood triglyceride level > 200 mg/dL prior to start of the ingestion of L-glutamine and a measured blood triglyceride level of <190 mg/dL after ingesting the L-glutamine for a period of at least one month.

6. L-glutamine for use according to claim 5, wherein the measured triglyceride blood level after ingesting the L-glutamine for a period of at least one month is ≤ 6.3 170 mg/dL.

7. L-glutamine for use according to claim 3, wherein the solution further comprises one or more buffers or flavorants.

8. L-glutamine or an L-glutamine salt for use according to any one of claims 1-7, wherein the L-glutamine or an L-glutamine salt is administered in conjunction with one or more of a statin, a fibrate, niacin, fish oil, or an omega-3-acid ethyl ester.

## Patentansprüche

1. L-Glutamin oder ein L-Glutaminsalz zur Verwendung bei der Behandlung von Hypertriglyceridämie, wobei 0,05 g/kg Körpergewicht bis 10,0 g/kg Körpergewicht des L-Glutamins oder des L-Glutaminsalzes pro Tag durch eine Person, die Diabetes hat, aufgenommen wird.

2. L-Glutamin zur Verwendung nach Anspruch 1.

3. L-Glutamin zur Verwendung nach Anspruch 2, wobei das L-Glutamin als Teil einer wässrigen Lösung aufgenommen wird.

4. L-Glutamin zur Verwendung nach Anspruch 3, wobei das L-Glutamin für einen Zeitraum von mindestens einem Monat aufgenommen wird.

5. L-Glutamin zur Verwendung nach Anspruch 4, wobei die Person, die Hypertriglyceridämie aufweist, einen gemessenen Blut-Triglycerid-Spiegel > 200 mg/dL vor Beginn der Aufnahme von L-Glutamin und einen gemessenen Blut-Triglycerid-Spiegel von ≤ 190 mg/dL nach Aufnahme des L-Glutamins für einen Zeitraum von mindestens einem Monat aufwies.

6. L-Glutamin zur Verwendung nach Anspruch 5, wobei der gemessene Triglycerid-Blutspiegel nach Aufnahme des L-Glutamins für einen Zeitraum von mindestens einem Monat ≤ 6,3 170 mg/dL ist.

7. L-Glutamin zur Verwendung nach Anspruch 3, wobei die Lösung ferner einen oder mehrere Puffer oder Geschmacksstoffe umfasst.

8. L-Glutamin oder ein L-Glutaminsalz zur Verwendung nach einem der Ansprüche 1-7, wobei das L-Glutamin oder ein L-Glutaminsalz in Verbindung mit einem oder mehreren von einem Statin, einem Fibrat, Niacin, Fischöl oder einem Omega-3-Säureethylester verabreicht wird.

## Revendications

1. L-glutamine ou sel de L-glutamine pour utilisation dans le traitement de l'hypertriglycéridémie, où 0,05 g/kg de poids corporel à 10,0 g/kg de poids corporel de la L-glutamine ou du sel de L-glutamine sont ingérés par jour par une personne souffrant de diabète.

2. L-glutamine pour utilisation selon la revendication 1.

3. L-glutamine pour utilisation selon la revendication 2, où la L-glutamine est ingérée en tant que partie d'une solution aqueuse.

4. L-glutamine pour utilisation selon la revendication 3, où la L-glutamine est ingérée pendant une période d'au moins un mois.

5. L-glutamine pour utilisation selon la revendication 4, où la personne souffrant d'hypertriglycéridémie avait un taux de triglycérides mesuré dans le sang > 200 mg/dl avant le début de l'ingestion de la L-glutamine et un taux de triglycérides mesuré dans le sang ≤ 190 mg/dl après l'ingestion de la L-glutamine pendant une période d'au moins un mois.

6. L-glutamine pour utilisation selon la revendication 5, où le taux sanguin de triglycérides mesuré après l'ingestion de la L-glutamine pendant une période d'au moins un mois est ≤ 6,3 170 mg/dl.

7. L-glutamine pour utilisation selon la revendication 3, où la solution comprend en outre un ou plusieurs tampons ou aromatisants.

8. L-glutamine ou sel de L-glutamine pour utilisation selon l'une quelconque des revendications 1 à 7, où la L-glutamine ou un sel de L-glutamine est administré conjointement avec un ou plusieurs d'une statine, d'un fibrate, d'une niacine, d'une huile de poisson ou d'un ester éthylique d'acide oméga-3.
